(19) Europäisches Patentamt
European Patent Office
Office européen des brevets

(11) **EP 3 884 261 B1**

(12) **EUROPÄISCHE PATENTSCHRIFT**

(45) Veröffentlichungstag und Bekanntmachung des Hinweises auf die Patenterteilung:
**28.12.2022 Patentblatt 2022/52**

(21) Anmeldenummer: **19761725.1**

(22) Anmeldetag: **06.08.2019**

(51) Internationale Patentklassifikation (IPC):
**G01N 11/14** $^{(2006.01)}$ **G01N 33/30** $^{(2006.01)}$

(52) Gemeinsame Patentklassifikation (CPC):
**G01N 33/30; G01N 11/142**

(86) Internationale Anmeldenummer:
**PCT/DE2019/100710**

(87) Internationale Veröffentlichungsnummer:
**WO 2020/103969 (28.05.2020 Gazette 2020/22)**

(54) **VERFAHREN ZUR BESTIMMUNG VON SCHMIERSTOFFEIGENSCHAFTEN**

METHOD FOR DETERMINING LUBRICANT PROPERTIES

PROCÉDÉ POUR DÉTERMINER DES PROPRIÉTÉS DE LUBRIFIANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.11.2018 DE 102018129457**

(43) Veröffentlichungstag der Anmeldung:
**29.09.2021 Patentblatt 2021/39**

(73) Patentinhaber: **Schaeffler Technologies AG & Co. KG**
**91074 Herzogenaurach (DE)**

(72) Erfinder: **BERG, Franziska**
**96173 Oberhaid (DE)**

(56) Entgegenhaltungen:
**EP-A1- 0 461 704      DE-A1- 10 350 554**
**DE-A1- 19 650 616      US-A1- 2014 193 110**

**Beschreibung**

Die Erfindung betrifft ein Verfahren zur Bestimmung von Schmierstoffeigenschaften. Insbesondere betrifft die Erfindung ein Verfahren zur Bestimmung der Eignung eines Schmierstoffs, speziell eines Schmierfetts zur Vermeidung von False-Brinelling-Schadensbildern (auch als eine spezielle Form der Riffelbildung bezeichnet) in fettgeschmierten Wälzlagern.

In Wälz- oder Kugellagern bilden Schmierstoffe einen Film zwischen Schmierflächen und verhindern so, dass ein direkter Kontakt zwischen sich gegeneinander bewegenden Oberflächen besteht. Auf diese Art werden mechanische Reibung und Verschleiß verringert. Die Eigenschaften von Schmierfetten in Bezug auf ihre Viskosität hängen dabei stark von dem Temperaturbereich ab, in welchem sie eingesetzt werden. Desgleichen hängt ihre Eignung als Schmierstoff davon ab, mit welcher Geschwindigkeit sich die Oberflächen zueinander bewegen. Bei höheren Geschwindigkeiten erhitzt sich der Schmierstoff stärker und kann eine Temperatur erreichen, bei der er sich zersetzt. Bei tiefen Temperaturen kann aufgrund der höheren Viskosität des Schmierstoffes das Nachfließen in den Zwischenraum zwischen den Oberflächen so langsam erfolgen, dass der Film zwischen den Schmierflächen reißt.

Die Schmierung von Wälzlagern soll vor allem zwei Ziele erfüllen: die Minimierung der Reibung und den Schutz vor Verschleiß. Während des normalen Betriebs von Wälzlagern wird zwischen unterschiedlichen Schmierungszuständen unterschieden, in denen sich, je nach Drehzahl des Lagers sogenannte "volltragende Schmierfilme" ausbilden können. Je nach Eigenschaft des Schmierstoffs wird die Bildung von volltragenden Schmierfilmen begünstigt. Einer der maßgeblichen Parameter ist dabei die Viskosität des Schmierstoffs. Eine besondere Problematik tritt auf, wenn neue, frisch mit Schmierstoff befüllte Wälzlager transportiert werden. Betroffen sind hiervon z. B. PKW-Radlager während des Fahrzeugtransportes auf Autoreisezügen oder auf LKW-Transportern. Hierbei kommt es, speziell bei den zweireihigen Schrägkugellagern, immer wieder zu Schadensbildern, welche an den Positionen der belasteten Kugeln Schäden an den Innen- und Außenringlaufbahnen zeigen. Diese Schäden an den Innen- und Außenringlaufbahnen können durchaus Tiefen von mehreren Mikrometern erreichen und erscheinen ohne nähere mikroskopische Untersuchung zunächst wie durch die Kugeln hervorgerufene Eindrückungen. Solche Schadensbilder können auf einem sogenannten "False-Brinelling" beruhen.

Die Anti-False-Brinelling-Eigenschaft von Schmierstoffen, also die Fähigkeit, den oben beschriebenen Schaden zu verhindern, ist bislang nur in zeitaufwendigen und teuren Prüfstandversuchen zu ermitteln. In einem rheologischen Prüfstand oder Messgerät werden Kennwerte von Schmierstoffen ermittelt. Als Labor-Prüfmethode werden im Wesentlichen zwei rheologische Verfahren zur Prüfung von Schmierstoffen in Anlehnung an DIN51810-2 herangezogen. Die DIN51810-2 beschreibt, wie das Messverfahren aufgebaut ist, welche Geräte verwendet werden und welche rheologischen Kennwerte durch die Messung ermittelt werden können.

Die Erfindung stellt sich daher die Aufgabe, ein einfaches und kostengünstiges Verfahren anzugeben, mit dem die Eignung eines Schmierstoffes, insbesondere eines Schmierfetts, zur Vermeidung von False-Brinelling-Schäden in einem Lager rasch bestimmt werden kann.

Die Aufgabe wird erfindungsgemäß gelöst durch ein Verfahren zur Bestimmung von Schmierstoffeigenschaften gemäß dem beigefügten Anspruch 1. Bevorzugte Ausführungsformen der Erfindung sind Gegenstand der Unteransprüche.

Untersuchungen haben gezeigt, dass typische False-Brinelling-Schäden keineswegs auf eine statische Überlastung des Laufbahnmaterials durch die Wälzkörper zurückzuführen sind. Vielmehr kommen diese Art von Schäden nur durch das Zusammenwirken mehrerer Einflussgrößen zustande. Diese sind ein hochbelasteter Punktkontakt im fettgeschmierten Wälzlager, ein grundsätzlich stillstehendes Lager, welches jedoch eine Mikrobewegung von etwa ± 1° in radialer Richtung erfährt und niedrige Außentemperaturen von -20°C und tiefer. Es wurde herausgefunden, dass False-Brinelling-Schäden insbesondere dann auftreten, wenn die Wälzkörper/Kugeln der Lastzone eines Lagers nicht nur den vorhandenen Schmierstoff sondern vor allem freies Grundöl des Schmierfettes aus dem Kontaktbereich verdrängen und dieses aufgrund der temperaturbedingt hohen Viskosität nicht schnell genug in den Kontaktbereich zurückfließen kann. Dadurch kann es so zu einem nahezu ungeschmierten Wälzkontakt kommen, wodurch Verschleiß und Korrosion auftreten können. Die so entstehenden Oberflächenschäden, die im Abstand der Wälzkörper auf den Lagerflächen entstehen, werden als False-Brinelling-Schäden bezeichnet und können durch eine veränderte Zusammensetzung der Schmierstoffe bzw. geeignete Auswahl der einzusetzenden Schmierstoffe reduziert werden.

Der Erfindungsgedanke beruht im wesentlichen auf der Erkenntnis, dass zu ermittelnde rheologische Kenngrößen eines untersuchten Schmierstoffes bei mindestens zwei unterschiedlichen, vorbestimmten Temperaturen eine Aussage über die Eignung des Schmierstoffes zur Vermeidung von False-Brinelling-Schäden in einem Lager in dem Temperaturbereich zwischen den beiden Temperaturen und ggfs. darüber hinaus ermöglichen. Dazu werden die gemessenen Kenngrößen bei den beiden Temperaturen miteinander in ein Wertefeld (Diagramm oder Wertetabelle) eingetragen, und aus der Lage des sich ergebenden Wertepaars in dem Wertefeld wird eine Angabe bzw. ein Signal generiert, welches repräsentativ ist für die Eignung des Schmierstoffes zur Vermeidung von False-Brinelling-Schäden in einem Lager. Im Sinne der vorliegenden Erfindung wird unter einem Schmierstoff bevorzugt ein Schmierfett verstanden, wie es typischerweise in Lagern verwendet wird.

Um die auf diese Art gewonnenen Ergebnisse mit Ergebnissen, wie sie in herkömmlichen Verfahren gewonnenen werden, vergleichen zu können, weist das erfindungsgemäße Verfahren vorzugsweise einen Umrechnungsschritt auf, in welchem Umrechnungsgrößen angewandt werden.

Das erfindungsgemäße Verfahren zum Bestimmen einer Eignung eines Schmierstoffes, insbesondere eines Schmierfetts, zur Vermeidung von False-Brinelling-Schäden in einem Lager weist die Schritte auf:

- erstes Konditionieren eines Rheometers mit den Teilschritten:

    a) Temperieren des Rheometers auf eine erste Temperatur,
    b) Setzen eines ersten Nullpunkts nach Ablauf einer vorgegebenen ersten Temperierzeit des Rheometers,

- erstes Befüllen des Rheometers mit einer Schmierstoffprobe,
- erstes Deformieren der Schmierstoffprobe und Ermitteln der ersten Schubspannung aus der Scherdeformation der Schmierstoffprobe bei der ersten Temperatur;
- zweites Konditionieren eines Rheometers mit den Teilschritten:

    c) Temperieren des Rheometers auf eine zweite Temperatur,
    d) Setzen eines zweiten Nullpunkts nach Ablauf einer vorgegebenen zweiten Temperierzeit des Rheometers,

- zweites Befüllen des Rheometers mit einer Schmierstoffprobe,
- zweites Deformieren der Schmierstoffprobe und Ermitteln der zweiten Schubspannung aus der Scherdeformation der Schmierstoffprobe bei der zweiten Temperatur,
- Klassifizieren des Schmierstoffes als geeignet oder nicht geeignet zur Vermeidung von False-Brinelling-Schäden in einem Lager in Abhängigkeit von der ermittelten ersten Schubspannung und der ermittelten zweiten Schubspannung.

Als aussagekräftige Temperaturwerte für die Beurteilung eines Schmierstoffes, insbesondere eines Schmierfetts, in Bezug auf seine Eignung zur Vermeidung von False-Brinelling-Schäden in einem Lager haben sich die im parktischen Einsatz des Schmierstoffes tiefste anzunehmende Temperatur und beispielsweise die übliche Temperatur beim Einbringen des Schmierstoffs in das Lager erwiesen. Daher wird im auszuführenden Verfahren die erste Temperatur bevorzugt im Bereich -45°C bis -5°C und die zweite Temperatur bevorzugt im Bereich +20°C bis +50°C gewählt. Insbesondere wird das Verfahren bei der ersten Temperatur von -30°C und bei der zweiten Temperatur von +25°C durchgeführt.

In zahlreichen Versuchen wurde herausgefunden, dass die Eignung des Schmierstoffes zur Vermeidung von False-Brinelling-Schäden in einem Lager sehr gut ist, wenn bei der ausgeführten Scherdeformation bei der ersten Temperatur die erste Schubspannung bis 1000 Pa beträgt und bei der zweiten Temperatur die zweite Schubspannung bis 100 Pa beträgt. Eine gute Eignung besteht, wenn die erste Schubspannung bis 1750 Pa beträgt und die zweite Schubspannung bis 275 Pa beträgt. Nur bedingt tauglich ist der Schmierstoff, wenn die erste Schubspannung bis 2500 Pa beträgt und die zweite Schubspannung bis 375 Pa beträgt. Schlecht geeignet unter dem Gesichtspunkt der Vermeidung von False-Brinelling-Schäden ist der Schmierstoff, wenn die erste Schubspannung über 2500 Pa beträgt und die zweite Schubspannung über 375 Pa beträgt.

Da bei den einzelnen Messungen Abweichungen auftreten, wird das Verfahren zur Verbesserung des statistischen Fehlers bevorzugt für jeden Messwert jeweils mit zwei, drei oder mehr Messungen durchgeführt.

Ein Vorteil der Erfindung besteht u.a. darin, dass das Verfahren schnell und ohne großen apparativen Aufwand durchgeführt werden kann, so dass eine schnelle Beurteilung eines Schmierstoffes insbesondere über seine Eignung als Schmierstoff zur Vermeidung von False-Brinelling-Schäden in einem Lager ermöglicht wird.

Weitere Merkmale und Vorteile der Erfindung ergeben sich aus der folgenden Beschreibung von bevorzugten Ausführungsformen der Erfindung, unter Bezugnahme auf die beigefügte Zeichnung. Es zeigen:

Fig. 1     ein Ablaufplan einer Ausführungsform des erfindungsgemäßen Verfahrens;

Fig. 2     ein Diagramm mit Messwerten, die in einer ersten Messung gewonnen wurden;

Fig. 3     ein Diagramm mit Messwerten, die in einer zweiten Messung gewonnen wurden;

Fig. 4     ein Diagramm mit Messwerten, die in einer dritten Messung gewonnen wurden:

Fig. 5    ein Diagramm mit Messwerten, die in einer vierten Messung gewonnen wurden.

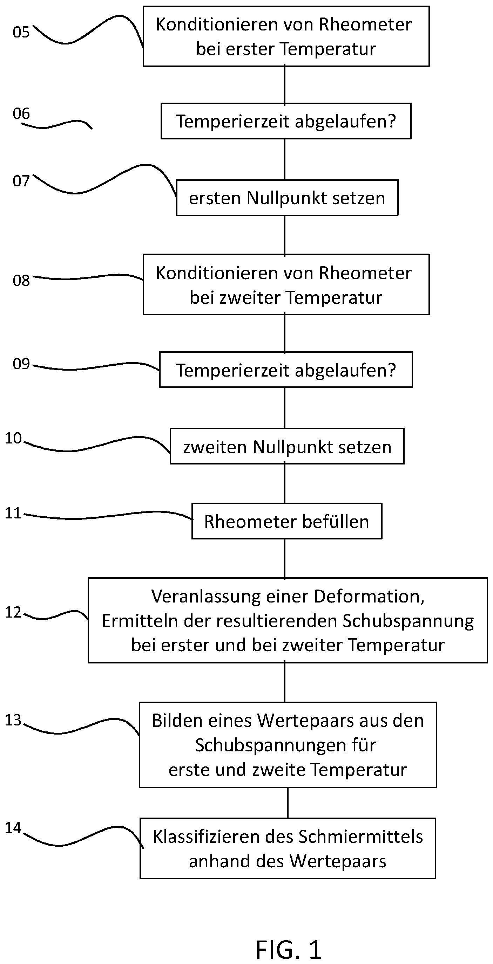

Fig. 1 zeigt einen Ablaufplan eines erfindungsgemäßen Verfahrens zum Bestimmen einer Eignung eines Schmierstoffes, insbesondere eines Schmierfetts, zur Vermeidung von False-Brinelling-Schäden in einem Lager mit folgenden Verfahrensschritten: In einem ersten Schritt 05 wird ein Rheometer zum Bestimmen der Fließeigenschaften eines Schmierstoffes bei einer ersten Temperatur konditioniert. Das Rheometer wird dazu auf die erste Temperatur gebracht. Auf dieser ersten Temperatur wird das Rheometer im Schritt 06 über eine vorgegebene Temperierzeit gehalten. In diesem Zeitraum relaxiert das Rheometer, d. h. in dem Rheometer soll nach der Temperierzeit kein Temperaturgradient mehr auftreten. Während dieser Temperierzeit (auch als Einschwingzeit oder Relaxationszeit bezeichnet) wird in Schritt 06 laufend überprüft, ob die Temperierzeit schon abgelaufen ist und der nächste Schritt eingeleitet werden kann.

Wenn die Temperierzeit abgelaufen ist, wird das Rheometer justiert, d. h. es wird in Schritt 07 ein erster Nullpunkt gesetzt, auf den sich die weiteren rheologischen Messungen bei der ersten Temperatur beziehen.

Die rheologische Messung beginnt damit, dass in Schritt 08 das Rheometer mit einer Schmierstoffprobe (Schmierfett) befüllt wird. In Schritt 09 wird eine vorbestimmte Scherdeformation auf die Schmierstoffprobe ausgeübt, so dass sich die Schmierstoffprobe in dem Rheometer deformiert. Neben der Größe der Scherdeformation und der dafür aufgewendeten Schubspannung werden weitere rheologische Größen des untersuchten Schmierstoffes wie z. B. das Speicher- und Verlustmodul bei der jeweiligen Messtemperatur ermittelt. Grundsätzlich sind dem Fachmann rheologische Messungen bekannt, sodass auf eine Erläuterung von Einzelheiten hier verzichtet werden kann.

Die Schmierstoffprobe wird nun aus dem Rheometer entfernt, um anschließend im Schritt 10 eine zweite Konditionierung bei einer zweiten Temperatur auszuführen. In Schritt 10 wird das Rheometer dafür auf die zweite Temperatur gebracht. In Schritt 11 wird das Einhalten einer zweiten Temperierzeit überwacht, und in Schritt 12 wird ein zweiter Nullpunkt gesetzt, auf den sich die weiteren rheologischen Messungen bei der zweiten Temperatur beziehen.

Nach dieser zweiten Konditionierung und einer erneuten Befüllung des Rheometers mit einer weiteren Probe desselben Schmierstoffs in Schritt 13, können die rheologischen Untersuchungen bei der zweiten Temperatur durchgeführt werden. In Schritt 14 wird eine vorbestimmte Scherdeformation auf die weitere Schmierstoffprobe ausgeübt und die Schubspannung ermittelt.

Die bestimmte Schubspannung bei der ersten Temperatur und die bestimmte Schubspannung bei der zweiten Temperatur werden beide für die nachfolgende Ermittlung der Eignung des Schmierstoffes zur Vermeidung von False-Brinelling-Schäden in einem Lager berücksichtigt. Zunächst wird die erfasste Scherdeformation in eine Scherrate umgerechnet und für diese wird die zugehörige Schubspannung bestimmt, woraufhin in Schritt 15 Wertepaare gebildet werden. Ein solches Wertepaar umfasst somit einen Schubspannungswert bei der ersten Temperatur und einen Schubspannungswert bei der zweiten Temperatur. Das Wertepaar definiert eine Position in einem zweidimensionalen Wertefeld, wobei diese Position ein Maß ist für die Eignung des Schmierstoffes zur Vermeidung von False-Brinelling-Schäden in einem Lager, in welchem der Schmierstoff eingesetzt werden soll.

Zum besseren Verständnis der Erfindung kann dieses Wertefeld als ein Diagramm dargestellt werden, in welchem die Wertepaare für unterschiedliche Schmierstoffe eingetragen werden, wobei auf der x-Achse die Schubspannung bei der ersten Temperatur aufgetragen ist und auf der y-Achse die Schubspannung bei der zweiten Temperatur aufgetragen ist.

Anhand des Wertepaars, also aus der Lage des Wertepaars in dem Wertefeld, wird in Schritt 16 die Eignung des Schmierstoffes zur Vermeidung von False-Brinelling-Schäden in einem Lager ermittelt, wobei die erste Schubspannung und die zweite Schubspannung in die Bewertung einfließen.

Nachfolgend werden konkrete Beispiele zur Aufnahme von Messwerten und deren Klassifizierung gemäß dem erfindungsgemäßen Verfahren erläutert.

Vorbereitungen und Messung bei -30 °C = erste Temperatur:

Zu Beginn wird das Rheometer inklusive Messsystem auf -30 °C temperiert. Nach Ablauf einer Temperierzeit von ca. 30 Minuten wird der erste Nullpunkt gesetzt. Da -30 °C weit unter Raumtemperatur liegt, wird das Rheometer nach dem Setzen des ersten Nullpunktes und vor dem Auftragen der Schmierstoffprobe erneut auf Raumtemperatur gebracht, um Kondensation von Wasserdampf und Reifbildung unterhalb von 0 °C zu vermeiden. Nachdem das Rheometer wieder auf Raumtemperatur gebracht wurde, wird es mit der Schmierstoffprobe befüllt. Dann wird das Messsystem mit der Schmierstoffprobe auf -30 °C heruntergekühlt. Wenn die -30 °C erreicht sind wird diese Temperatur für ca. 30 Minuten konstant gehalten um sicherzustellen, dass das Messsystem wie auch die Schmierstoffprobe tatsächlich die erste Temperatur von hier -30 °C haben. Nun wird ein Amplituden -Sweep zur Bestimmung der Schubspannung bei der ersten Temperatur ausgeführt. Dabei wird die Scherdeformation von 0,01 % auf 1000 % gesteigert. Der erste Teil der Messung ist damit beendet, sodass das Rheometer entleert und gesäubert werden kann.

Vorbereitungen und Messung bei +25 °C = zweite Temperatur

Zu Beginn wird das Rheometer inklusive Messsystem auf +25 °C temperiert. Nach Ablauf einer Temperierzeit von ca. 5 Minuten wird der zweite Nullpunkt gesetzt. Das Rheometer wird dann erneut mit einer Probe desselben Schmierstoffs befüllt. Das Messsystem und die Probe werden auf +25 °C temperiert und anschließend wird ein zweiter Amplituden-Sweep zur Bestimmung der zweiten Schubspannung ausgeführt. Dabei wird die Scherdeformation von 0,01 % auf 1000 % gesteigert. Damit ist die die Messung beendet und die Messwerte werden weiter verarbeitet.

In dem zuvor beschriebenen Beispiel erfolgt die Bestimmung der rheologischen Größen einmal bei -30°C (erste Temperatur) und einmal bei +25°C (zweite Temperatur) in einem Amplituden-Sweep. In dem Amplituden-Sweep wird die Scherdeformation des Schmierstoffes zwischen 0,01% und 1000% verändert. Die Amplitude wird mit einer konstanten Frequenz von 10 rad/s verändert und nimmt dabei in Zehnerpotenzen zu. Die Messwerte können dabei beispielsweise mit 5 oder 10 oder 20 Punkten pro Dekade aufgenommen werden. Die angewendete Scherdeformation wird im Rahmen der Messung vorgegeben. Aus dieser Vorgabe und dem Messwert kann in an sich bekannter Weise unter Berücksichtigung weiterer Gerätekonstanten und Materialwerte die Schubspannung bestimmt werden.

Dazu wird bei der Auswertung des Amplituden-Sweep bei +25°C anhand der Messdaten der Schubspannungswert bei einer Scherrate 0,36 s$^{-1}$ bestimmt. Bei dem Amplituden-Sweep bei -30°C wird die aufgezeichnete Scherdeformation in gleicher Weise zunächst in eine Scherrate umgerechnet und für diese wird nachfolgend ein Schubspannungswert ermittelt, beispielsweise aus einer hinterlegten Wertetabelle oder mithilfe eines implementierten mathematischen Zusammenhangs. Aus dem Kennwert bei +25°C und dem Kennwert bei -30°C wird ein Wertepaar gebildet. Dieses Wertepaar definiert eine Position in einem Wertefeld, wobei das Wertefeld z. B. in einem Speicher abgelegt sein kann oder auch durch eine mathematische Funktion beschreibbar ist, welche durch eine Software ausführbar ist. Zur Verdeutlichung kann das Wertepaar auch automatisiert in einem Diagramm bildlich eingetragen werden, welches beispielsweise zur Anzeige gebracht oder ausgedruckt wird.

Die Position des Wertepaars im Wertefeld ist ein Maß dafür, ob der untersuchte Schmierstoff gute oder schlechte Anti-False-Brinelling-Eigenschaften aufweist. Des Weiteren ist aus dem Wertepaar der beiden Schubspannungswerte nicht nur ermittelbar, ob gute oder schlechte Anti-False-Brinelling-Eigenschaften vorliegen, sondern auch in welchem Maße.

Mit dem erfindungsgemäßen Verfahren können Schmierstoffe, insbesondere Schmierfette, deren Anti-False-Brinelling-Eigenschaften auf gute Fließeigenschaften zurückzuführen sind, untersucht werden. Zur Bestimmung der Anti-False-Brinelling-Eigenschaften bis -30°C eines Fettes mittels rheologischem Verfahren wird das Messsystem bevorzugt zunächst mit technischem Ethanol, Brennspiritus, Isopropanol, technischem Aceton oder Spezialbenzin gereinigt. Das Messsystem umfasst ein Rheometer mit Peltierplatte, ein Platte-Messsystem für Rheometer, vorzugsweise mit einer Größe von Ø 25 mm. Zusätzlich zur Peltierplatte muss auch eine temperierte Abdeckhaube für das Messsystem eingesetzt werden, um das Messsystem auf die gewünschte Temperatur zu bringen. Bei Einsatz eines Peltierelements muss dieses gegengekühlt werden. Hierfür wird vorzugsweise ein Kryostat bzw. Thermostat eingesetzt. Nach der Reinigung wird das Messsystem mit einer Schmierstoffprobe befüllt, beispielsweise unter Verwendung eines Spatels.

Beim Befüllen des Messsystems wird jeweils eine ausreichende Menge des Schmierstoffes von ca. 0,5 g mit kleinem Überschuss mittig auf die untere Platte aufgebracht. Anschließend wird die obere Platte auf einen Abstand von 1,025 mm, die sog. Trimm-Position, zur unteren Platte herabgesenkt und überständiges Schmierstoffe mit Hilfe eines Spatels entfernt. Danach kann die finale Messposition von 1,000 mm angefahren werden. Während des Trimmens darf kein Schmierstoffe aus dem Messspalt entfernt werden, ansonsten ist die Befüllung des Spaltes nicht korrekt und führt zu einer Verfälschung der Messwerte.

Die eigentliche Messung erfolgt bei der jeweiligen Temperatur. Es ist verständlich, dass es irrelevant ist, ob die niedrigere oder die höhere Temperatur als erste Messtemperatur gewählt wird. Vorzugsweise werden pro Messtemperatur zwei oder mehr Messungen durchgeführt. Auf diese Art lässt sich der statistische Fehler der Messungen verringern.

Zur Auswertung der Messungen und als Aussage über die Eignung des Schmierfetts als Anti-False-Brinelling-Schmierstoff werden die Messwerte der Scherdeformation in Scherraten umgerechnet. Zur Bestimmung des Kennwertes des untersuchten Schmierstoffes werden die Messwerte für die Scherdeformation y nach der folgenden Gleichung in eine Scherrate dy/dt umgerechnet:

$$\text{d}\gamma/\text{dt (berechnet)} = \gamma \text{ (gemessen)} * 0,071$$

Anschließend wird vorzugsweise derjenige Messwert gesucht, bei dem die berechnete Scherrate 0,36 s$^{-1}$ beträgt, und der dazugehörige Schubspannungswert wird abgelesen bzw. automatisiert aus einem Wertefeld ermittelt.

Die beiden ermittelten Kennwerte werden als Wertepaar zusammengefasst und für die Bestimmung der Eignung als Anti-False-Brinelling-Schmierstoff herangezogen, indem die Position des Wertepaares in einem Wertefeld ausgewertet wird. Diese Auswertung kann durch Vergleich des Wertepaares mit einem Wertefeld erfolgen, welches in einem Speicher abgelegt ist, oder anhand eines mathematischen Zusammenhangs, der über eine Software abgebildet werden kann.

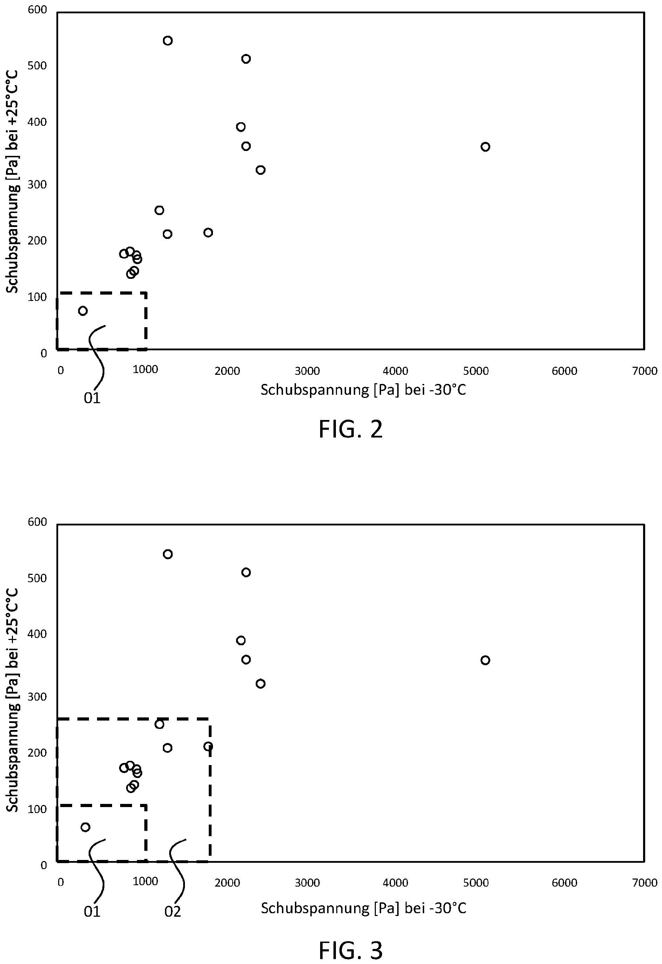

Diese Bestimmung der Position des Wertepaares lässt sich anhand von Diagrammen verdeutlichen, wie sie in den Fig. 2 bis 5 dargestellt ist. In Fig. 2 ist als horizontale Achse der Kennwert des Schmierfetts bei -30°C aufgetragen, und als vertikale Achse ist der Kennwert des Schmierfetts bei +25°C aufgetragen. Je nach Lage des Wertepaares in dem Diagramm bzw. Wertefeld wird die Anti-False-Brinelling-Eigenschaft des Fettes als sehr gut, gut, grenzwertig oder nicht geeignet klassifiziert.

In den Fig. 2 bis 5 sind die gemessenen Wertepaare jeweils als offene Kreise im Diagramm dargestellt. Dargestellt sind auch die jeweiligen Bereichsgrenzen für die Klassifizierung. In Fig. 2 fällt ein Wertepaar in einen inneren Bereich 1, der Wertepaare bis 1000 Pa bei -30°C und 100 Pa bei +25°C enthält. Schmierstoffe, deren Wertepaare in diesen Bereich 1 fallen, werden als "sehr gut" für die Vermeidung von False-Brinelling-Schäden klassifiziert.

Fig. 3 zeigt zusätzlich zu dem Bereich 1 mit den Bereichsgrenzen 1000 Pa bei -30°C und 100 Pa bei +25°C einen zweiten Bereich 2 mit den Bereichsgrenzen 1750 Pa bei -30°C und 275 Pa bei +25°C. Schmierstoffe, deren Wertepaare in diesen Bereich 2 fallen, werden als "gut" für die Vermeidung von False-Brinelling-Schäden klassifiziert. In dem in Fig. 3 gezeigten Beispiel erfüllen neun Wertepaare diese Bedingung.

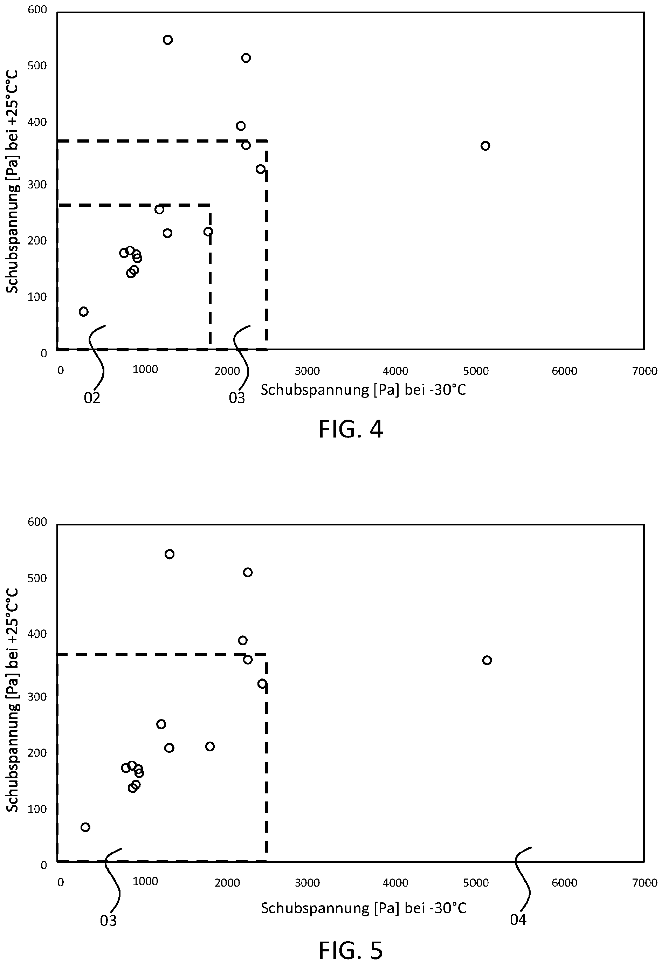

Fig. 4 zeigt einen dritten Bereich 3 mit den Bereichsgrenzen 2500 Pa bei -30°C und 375 Pa bei +25°C. Schmierstoffe, deren Wertepaare in diesen Bereich 3 fallen, werden als "noch bedingt tauglich" für die Vermeidung von False-Brinelling-Schäden klassifiziert. In dem gezeigten Beispiel erfüllen zwei Wertepaare diese Bedingung.

Fig. 5 zeigt als einen vierten Bereich 4 den Bereich über 2500 Pa bei -30°C und 375 Pa bei +25°C. Schmierstoffe, deren Wertepaare in diesen Bereich fallen, werden als "nicht ausreichend" für die Vermeidung von False-Brinelling-Schäden klassifiziert. In dem gezeigten Beispiel sind dies vier Wertepaare.

**Bezugszeichenliste**

01    Erster Bereich = "sehr gut"
02    Zweiter Bereich = "gut"
03    Dritter Bereich = "noch bedingt tauglich"
04    Vierter Bereich = "nicht ausreichend"

05 - 14    Verfahrensschritte

**Patentansprüche**

1. Verfahren zum Bestimmen der Eignung eines Schmierstoffes, insbesondere eines Schmierfetts, zur Vermeidung von False-Brinelling-Schäden in einem Lager, folgende Schritte umfassend:

   - erstes Konditionieren eines Rheometers mit den Teilschritten:

      a) Temperieren (5, 6) des Rheometers auf eine erste Temperatur,
      b) Setzen (7) eines ersten Nullpunkts nach Ablauf einer vorgegebenen ersten Temperierzeit des Rheometers,

   - Befüllen (08) des Rheometers mit einer Schmierstoffprobe,
   - Deformieren (09) der Schmierstoffprobe und Ermitteln einer ersten Schubspannung aus der Scherdeformation der Schmierstoffprobe bei der ersten Temperatur, unter Bezug auf den ersten Nullpunkt des Messsystems,
   - zweites Konditionieren des Rheometers mit den Teilschritten:

      c) Temperieren (10, 11) des Rheometers auf eine zweite Temperatur,
      d) Setzen (12) eines zweiten Nullpunkts nach Ablauf einer vorgegebenen zweiten Temperierzeit des Rheometers,

   - erneutes Befüllen (13) des Rheometers mit einer Schmierstoffprobe,
   - Deformieren (14) der Schmierstoffprobe und Ermitteln einer zweiten Schubspannung aus der Scherdeformation der Schmierstoffprobe bei der zweiten Temperatur unter Bezug auf den zweiten Nullpunkt des Messsystems,
   - Klassifizieren (15, 16) des Schmierstoffes als geeignet oder nicht geeignet zur Vermeidung von False-Brinelling-Schäden in Abhängigkeit von der ermittelten ersten Schubspannung und der ermittelten zweiten Schubspannung.

2. Verfahren nach Anspruch 1, wobei die erste Temperatur bei -30°C und die zweite Temperatur bei +25°C gewählt wird.

3. Verfahren nach Anspruch 2, bei dem die Anti-False-Brinelling-Eigenschaft des Schmierstoffes in einem ersten Bereich (1) bis zu einer ersten Schubspannung bis 1000 Pa und bis zu einer zweiten Schubspannung bis 100 Pa als "sehr gut" klassifiziert wird, in einem zweiten Bereich (2) bis zu einer ersten Schubspannung bis 1750 Pa und bis zu einer zweiten Schubspannung bis 275 Pa als "gut" klassifiziert wird, in einem dritten Bereich (3) bis zu einer ersten Schubspannung bis 2500 Pa und bis zu einer zweiten Schubspannung bis 375 Pa als "bedingt tauglich" klassifiziert wird, und in einem vierten Bereich (4) über einer ersten Schubspannung von 2500 Pa und über einer zweiten Schubspannung von 375 Pa als "schlecht" klassifiziert wird.

4. Verfahren nach einem der Ansprüche 1 bis 3, wobei zur Verbesserung des statistischen Fehlers für jeden Messwert jeweils zwei Messungen durchgeführt werden.

5. Verfahren nach einem der Ansprüche 1 bis 4, wobei das erste und zweite Konditionieren des Rheometers jeweils bei geleertem Rheometer ausgeführt wird.

6. Verfahren nach einem der Ansprüche 1 bis 5, wobei der Teilschritt des Temperierens des Rheometers auf die erste Temperatur und die zweite Temperatur mit Hilfe einer Peltierplatte und einer Abdeckhaube erfolgt.

7. Verfahren nach einem der Ansprüche 1 bis 6, wobei der Schritt des Deformierens (09; 14) der Schmierstoffprobe als ein Amplituden-Sweep erfolgt, bei dem

  ○ die Scherdeformation mit einer konstanten Frequenz von 10 rad / s verändert wird und
  ○ die Scherdeformation von 0,01% auf 1000% verändert wird, wobei die Maximalamplitude innerhalb von 10 Punkten jeweils um den Faktor Zehn vergrößert wird.

8. Verfahren nach einem der Ansprüche 1 bis 7, wobei der Teilschritt des Temperierens des Rheometers mit einer vorgegebenen Heizrate über eine vorgegebene Dauer erfolgt.

9. Verfahren nach einem der Ansprüche 1 bis 8, wobei der Schritt des Ermitteins der Schubspannung aus der Deformation durch Umrechnen einer Scherdeformation y in eine Scherrate dy/dt und Ermitteln des Schubspannungswertes aus der Scherrate erfolgt.

**Claims**

1. A method for determining the suitability of a lubricant, in particular a lubricating grease, for avoiding false brinelling damage in a bearing, comprising the following steps:

  - a first conditioning of a rheometer, having the sub-steps:

    a) tempering (5, 6) the rheometer to a first temperature,
    b) setting (7) a first zero point after a predetermined first tempering time of the rheometer,

  - filling (08) the rheometer with a lubricant sample,
  - deforming (09) the lubricant sample and determining a first shear stress from the shear deformation of the lubricant sample at the first temperature, with reference to the first zero point of the measuring system,
  - a second conditioning of the rheometer, having the sub-steps:

    c) tempering (10, 11) the rheometer to a second temperature,
    d) setting (12) a second zero point after a predetermined second tempering time of the rheometer,

  - refilling (13) the rheometer with a lubricant sample,
  - deforming (14) the lubricant sample and determining a second shear stress from the shear deformation of the lubricant sample at the second temperature, with reference to the second zero point of the measuring system,
  - classifying (15, 16) the lubricant as suitable or unsuitable for avoiding false brinelling damage depending on the determined first shear stress and the determined second shear stress.

2. The method according to claim 1, wherein the first temperature is selected as -30°C and the second temperature is selected as +25°C.

3. The method according to claim 2, in which the anti-false brinelling property of the lubricant is classified as "very good" in a first range (1) up to a first shear stress of 1000 Pa and up to a second shear stress of 100 Pa, is classified as "good" in a second range (2) of up to a first shear stress of 1750 Pa and up to a second shear stress of 275 Pa, is classified as "suitable to a limited extent" in a third range (3) of up to a first shear stress of 2500 Pa and up to a second shear stress of 375 Pa, and is classified as "poor" in a fourth range (4) above a first shear stress of 2500 Pa and above a second shear stress of 375 Pa.

4. The method according to any one of claims 1 to 3, wherein two measurements are carried out for each measured value in order to reduce statistical errors.

5. The method according to any one of claims 1 to 4, wherein the first and second conditioning of the rheometer are each carried out with the rheometer empty.

6. The method according to any one of claims 1 to 5, in which the sub-step of tempering the rheometer to the first temperature and the second temperature is carried out with the aid of a Peltier plate and a covering hood.

7. The method according to any one of claims 1 to 6, in which the step of deforming (09; 14) the lubricant sample takes place as an amplitude sweep in which

   ○ the shear deformation is changed with a constant frequency of 10 rad/s and
   ○ the shear deformation is changed from 0.01% to 1000%, wherein the maximum amplitude is increased by a factor of ten within 10 points.

8. The method according to any one of claims 1 to 7, wherein the sub-step of tempering the rheometer at a predetermined heating rate takes place over a predetermined duration.

9. The method according to any one of claims 1 to 8, wherein the step of determining the shear stress from the deformation is carried out by converting a shear deformation y into a shear rate dy/dt and determining the shear stress value from the shear rate.


**Revendications**

1. Procédé pour déterminer l'adéquation d'un lubrifiant, en particulier d'une graisse lubrifiante, pour éviter des dommages par faux effet Brinell dans un roulement, comprenant les étapes suivantes :

   - premier conditionnement d'un rhéomètre avec les sous-étapes :

      a) équilibrage de la température (5, 6) du rhéomètre à une première température,
      b) réglage (7) d'un premier point zéro après écoulement d'un premier temps d'équilibrage de la température prédéfini du rhéomètre,

   - remplissage (08) du rhéomètre avec un échantillon de lubrifiant,
   - déformation (09) de l'échantillon de lubrifiant et détermination d'une première contrainte de cisaillement à partir de la déformation par cisaillement de l'échantillon de lubrifiant à la première température, en référence au premier point zéro du système de mesure,
   - second conditionnement du rhéomètre avec les sous-étapes :

      c) équilibrage de la température (10, 11) du rhéomètre à une seconde température,
      d) réglage (12) d'un second point zéro après écoulement d'un second temps d'équilibrage de la température prédéfini du rhéomètre,

   - rechargement (13) du rhéomètre avec un échantillon de lubrifiant,
   - déformation (14) de l'échantillon de lubrifiant et détermination d'une seconde contrainte de cisaillement à partir de la déformation par cisaillement de l'échantillon de lubrifiant à la seconde température en référence au second point zéro du système de mesure,

- classement (15, 16) du lubrifiant comme approprié ou non pour éviter les dommages par faux effet Brinell en fonction de la première contrainte de cisaillement déterminée et de la seconde contrainte de cisaillement déterminée.

2. Procédé selon la revendication 1, la première température étant choisie à -30 °C et la seconde température à +25 °C.

3. Procédé selon la revendication 2, dans lequel la propriété anti-faux effet Brinell du lubrifiant est classée « très bonne » dans une première plage (1) jusqu'à une première contrainte de cisaillement allant jusqu'à 1000 Pa et jusqu'à une seconde contrainte de cisaillement allant jusqu'à 100 Pa, est classée comme « bonne » dans une deuxième plage (2) jusqu'à une première contrainte de cisaillement allant jusqu'à 1750 Pa et jusqu'à une seconde contrainte de cisaillement allant jusqu'à 275 Pa, est classée comme « appropriée sous conditions » dans une troisième plage (3) jusqu'à une première contrainte de cisaillement jusqu'à 2500 Pa et jusqu'à une seconde contrainte de cisaillement jusqu'à 375 Pa et est classée comme « mauvaise » dans une quatrième plage (4) au-dessus d'une première contrainte de cisaillement de 2500 Pa et au-dessus d'une seconde contrainte de cisaillement de 375 Pa.

4. Procédé selon l'une quelconque des revendications 1 à 3, deux mesures étant effectuées pour chaque valeur mesurée afin de rectifier l'erreur statistique.

5. Procédé selon l'une quelconque des revendications 1 à 4, les premier et second conditionnements du rhéomètre étant chacun effectués avec le rhéomètre vidé.

6. Procédé selon l'une quelconque des revendications 1 à 5, l'étape partielle d'équilibrage de la température du rhéomètre à la première température et à la seconde température étant réalisée à l'aide d'une plaque Peltier et d'une hotte de recouvrement.

7. Procédé selon l'une quelconque des revendications 1 à 6, l'étape de déformation (09 ; 14) de l'échantillon de lubrifiant se déroulant sous la forme d'un balayage d'amplitude dans lequel

   ○ la déformation par cisaillement est modifiée avec une fréquence constante de 10 rad/s et
   ○ la déformation par cisaillement passe de 0,01 % à 1000 %, l'amplitude maximale étant augmentée d'un facteur dix en 10 points.

8. Procédé selon l'une quelconque des revendications 1 à 7, l'étape partielle d'équilibrage de la température du rhéomètre à une vitesse de chauffage prédéfinie se déroulant sur une durée prédéfinie.

9. Procédé selon l'une quelconque des revendications 1 à 8, l'étape de détermination de la contrainte de cisaillement à partir de la déformation étant réalisée en convertissant une déformation par cisaillement y en un taux de cisaillement $dy/dt$ et en déterminant la valeur de contrainte de cisaillement à partir du taux de cisaillement.

FIG. 1

FIG. 2

FIG. 3

FIG. 4

FIG. 5